(19) Europäisches Patentamt
European Patent Office
Office européen des brevets

(11) **EP 1 757 975 B1**

(12) **EUROPÄISCHE PATENTSCHRIFT**

(45) Veröffentlichungstag und Bekanntmachung des Hinweises auf die Patenterteilung:
**08.10.2014 Patentblatt 2014/41**

(51) Int Cl.:
***G02C 7/04*** *(2006.01)*     ***A61F 9/00*** *(2006.01)*

(21) Anmeldenummer: **05018424.1**

(22) Anmeldetag: **24.08.2005**

(54) **Formstabile Kontaktlinse**

Rigid contact lens

Lentille de contacte rigide

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**28.02.2007 Patentblatt 2007/09**

(73) Patentinhaber: **Hecht Contactlinsen GmbH**
**79280 Au (DE)**

(72) Erfinder: **Muckenhirn, Dieter**
**79299 Wittnau (DE)**

(74) Vertreter: **Hofer, Dorothea et al**
**Prüfer & Partner GbR**
**Patentanwälte**
**Sohnckestrasse 12**
**81479 München (DE)**

(56) Entgegenhaltungen:
| | |
|---|---|
| WO-A-95/07487 | US-A- 5 347 326 |
| US-A- 5 695 509 | US-A1- 2002 159 025 |
| US-A1- 2003 175 259 | US-A1- 2004 257 524 |

**Beschreibung**

Die vorliegende Erfindung betrifft eine formstabile Kontaktlinse und im Speziellen eine Kontaktlinse zur Korrektur von visuellen Seheinbußen bei Keratokonus und anderen Fällen, sowie zur Überbrückung bei zentralen Epitheldefekten der Hornhaut.

In der Technik sind verschiedene Verfahren der Korrektur von Seheinbußen bekannt. Zu den häufigsten Verfahren gehören das Anpassen von Brillen und das Anpassen von Kontaktlinsen.

Es gibt Menschen in der Bevölkerung, die unter Keratokonus leiden, einer veränderten Struktur des Hornhautgewebes, bei der eine Vorwölbung von Hornhautanteilen auftritt. Es gibt weiterhin auch Menschen, die aufgrund anderer Umstände unter einer zentralen Narbenbildung der Hornhaut leiden, die zu einem permanenten zentralen Epitheldefekt führt. Bei diesen Menschen ist eine Korrektur der Seheinbußen mit Brillengläsern meist nicht in einem zufriedenstellenden Maße möglich. Häufig werden zur Korrektur der Seheinbußen daher Kontaktlinsen verwendet.

Die Verwendung von Kontaktlinsen ist jedoch ebenfalls nicht problemlos, da meist auf die irreguläre Hornhautoberfläche eine rotationssymmetrische oder torische Fläche aufgelegt wird. Dabei tritt insbesondere bei zu flach angepassten herkömmlichen Kontaktlinsen häufig das Problem auf, dass ein hoher mechanischer Druck auf die irreguläre Hornhautoberfläche ausgeübt wird. Dies kann zu einer Verschlechterung des Hornhautzustands führen und wird von den Trägern der Kontaktlinsen auch als schmerzhaft empfunden. Die bekannten Kontaktlinsen werden üblicherweise nach einer Dreipunkt-Auflagemethode oder nach einer Konturtechnik mit Apexüberbrückung angepasst.

Um das Problem des hohen mechanischen Drucks auf die irreguläre Hornhautoberfläche zu umgehen, werden Kontaktlinsen verwendet, die auf der dem Auge zugewandten Rückfläche in der zentralen optischen Zone eine sphärische Fläche mit einem kleineren Radius als dem der irregulären Hornhautoberfläche aufweisen. Eine solche Kontaktlinse ist zum Beispiel in der EP 0 235 328 B1 beschrieben. Es wird eine Überbrückung der sensiblen zentralen Hornhautzone erreicht. Bei diesem Vorgehen ist es jedoch nachteilig, dass als Folge der zum Teil sehr ausgeprägten Radienverkleinerung der zentralen optischen Zone auf der dem Auge zugewandten Rückfläche die Kontaktlinse in ihrer optischen Wirkung zum Stärkenausgleich erheblich stärker werden muss. Als Beispiel ändert sich die Stärke einer vollkorrigierenden Kontaktlinse mit dem Radius $r_0 = 6{,}5$ mm und -10.00 dpt bei einer Radienänderung auf $r_0 = 6{,}00$ mm auf -14.25 dpt. Obwohl eine solche steilere Linse die gleiche optische Wirkung auf dem Auge besitzt, wird der Visus des Kontaktlinsenträgers deutlich schlechter, wie in der Literatur beschrieben ist (Zadnik, K.; Mutti, D.O.; American Journal of Optometry and Physiological Optics, 9/1987, 698ff.). Häufig wird von dem Kontaktlinsenträger sogar ein höherer Dioptriewert als der gewünscht, der sich durch die Radienänderung rechnerisch ergibt.

Aus der EP 0 722 573 B1 ist eine Kontaktlinse bekannt, die einen sphärischen zentralen optischen Abschnitt auf der dem Auge zugewandten Seite aufweist, an den sich ein asphärischer peripherer Bereich anschließt, der an die Form der Hornhaut angepasst ist. Bei dieser Kontaktlinse ergeben sich die oben beschriebenen Probleme.

WO 95/07487 A offenbart eine gattungsgemäße formstabile Kontaktlinse zur Korrektur bei irregulär gekrümmter Hornhaut mit einer Vorderfläche und einer Rückfläche, die aus einer zentralen optischen Zone und einer peripheren Zone besteht, wobei die zentrale optische Zone einen Durchmesser und ein Zentrum hat, durch das eine zentrale optische Achse verläuft.

Es ist Aufgabe der vorliegenden Erfindung, eine Kontaktlinse zu schaffen, mit der ermöglicht ist, die Belastung einer irregulären Hornhautoberfläche zu verringern und gleichzeitig eine gute bzw. verbesserte Sehschärfe zu erreichen.

Die Aufgabe wird durch eine formstabile Kontaktlinse gemäß Anspruch 1 gelöst. Weiterbildungen der Erfindung sind in den Unteransprüchen gekennzeichnet.

Mit der erfindungsgemäßen Kontaktlinse ist erreicht, dass die Scheiteltiefe der zentralen optischen Zone verglichen zu einer herkömmlichen sphärischen oder torischen zentralen optischen Zone erhöht ist, sodass ein irregulärer Bereich der Hornhaut überbrückt werden kann, und dabei gleichzeitig ein zentraler Krümmungsradius im Zentrum der zentralen optischen Zone wie bei einer Kontaktlinse für eine reguläre Hornhaut gewählt werden kann. Somit muss, wenn ein irregulärer Bereich der Hornhaut überbrückt werden soll, die Stärke der Kontaktlinse nicht erhöht werden.

Der erfindungsgemäße Aufbau ermöglicht somit, dass die Rückfläche der Kontaktlinse so angepasst wird, dass sie der Kontur einer irregulären Hornhaut gerecht wird. Damit wird die mechanische Druckbelastung von dieser vermindert oder beseitigt und die Kontaktlinse stellt eine geringstmögliche physiologische Belastung dar. Folglich verschlechtert sich die visuelle Leistungsfähigkeit des Auges durch das Tragen der Kontaktlinse auch bei einem fortgeschrittenen Stadium eines Keratokonus nicht, sondern wird konstant gehalten oder verbessert.

Ferner kann die zentrale optische Zone je nach Anforderung rotationssymmetrisch oder nicht-rotationssymmetrisch sein.

Weitere Merkmale und Zweckmäßigkeiten der Erfindung ergeben sich aus der Beschreibung von Ausführungsbeispielen anhand der beigefügten Zeichnungen. Von den Figuren zeigen:

Fig. 1      eine schematische Schnittansicht einer Kontaktlinse gemäß einer Ausführungsform;

Fig. 2         eine schematische Schnittansicht der Kontaktlinse aus Fig. 1 auf einem Auge mit einer irregulären Hornhaut platziert;

Fig. 3a bis 3d     schematische Darstellungen zur Erläuterung des Krümmungsverlaufs der zentralen optischen Zone der Kontaktlinse aus Fig. 1 gemäß einer beispielhaften Ausführungsform.

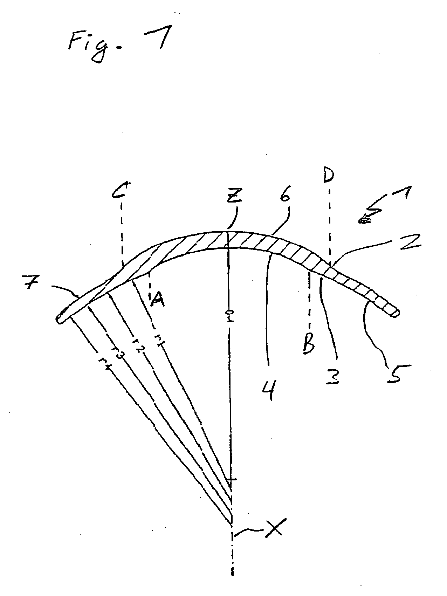

Im Folgenden wird mit Bezug auf Fig. 1 eine erste Ausführungsform der vorliegenden Erfindung beschrieben.

Wie in Fig. 1 dargestellt ist, weist die Kontaktlinse 1 eine Vorderfläche 2, die bei der Benutzung der Kontaktlinse von dem Auge des Trägers abgewandt ist, und eine Rückfläche 3 auf, die bei der Benutzung dem Auge des Trägers zugewandt ist. Die Rückfläche 3 besteht aus einer zentralen optischen Zone 4 zwischen den Linien A und B und zumindest einem peripheren Bereich 5, der sich außen ringsum an die zentrale optische Zone 4 anschließt. Die Vorderfläche 2 weist einen zentralen optischen Bereich 6 zwischen den Linien C und D und einen Randbereich 7 auf, der sich außen an den zentralen optischen Bereich 6 anschließt. Die optischen Eigenschaften der Kontaktlinse 1 sind im wesentlichen durch die zentrale optische Zone 4 und den zentralen optischen Bereich 6 bestimmt, wohingegen der periphere Bereich 5 und der Randbereich 7 hauptsächlich einem optimierten Sitz auf dem Auge des Kontaktlinsenträgers dienen. Der periphere Bereich 5 und der Randbereich 7 können jeweils durch mehrere aneinander anschließende Bereiche gebildet sein, die wie in Fig. 1 dargestellt ist, verschiedene Krümmungsradien $r_1$, $r_2$, $r_3$, $r_4$ etc. aufweisen. Die Kontaktlinse weist ferner ein Zentrum Z auf, durch das eine zentrale optische Achse X verläuft.

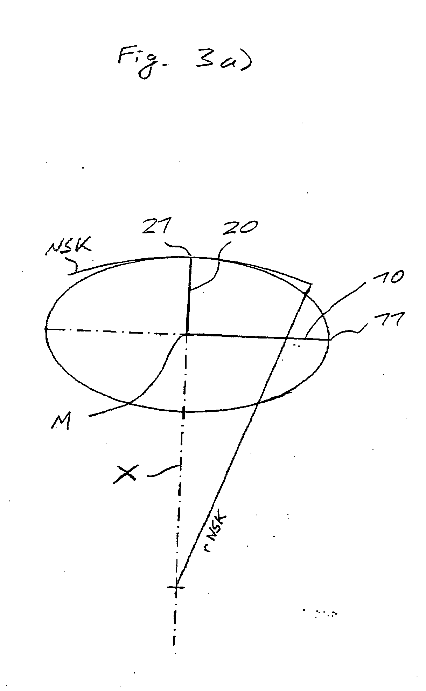

Im Folgenden wird die Geometrie der zentralen optischen Zone anhand der schematischen Darstellungen der Fig. 3a bis 3d beschrieben.

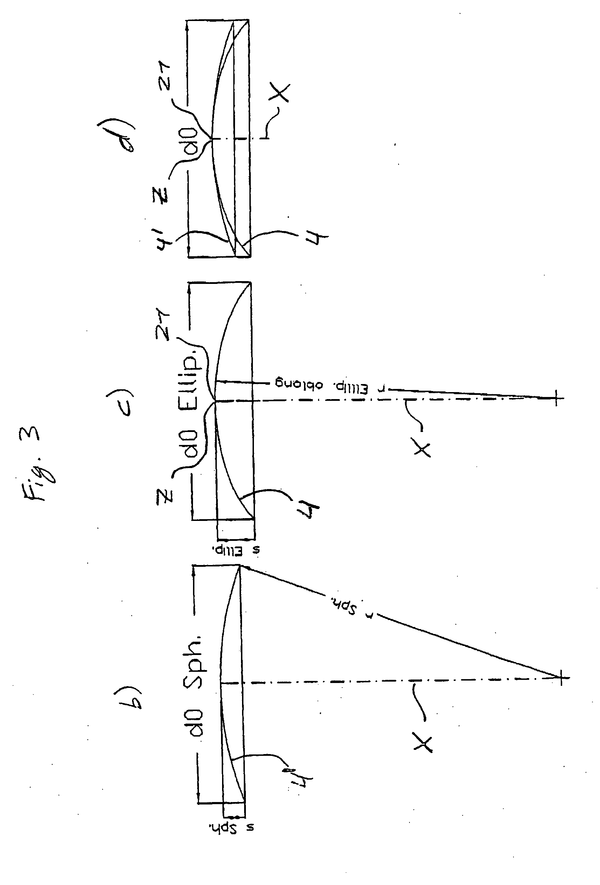

Zunächst wird anhand der Fig. 3b der Krümmungsverlauf der bekannten Kontaktlinsen beschrieben. Bei den bekannten Kontaktlinsen weist die zentrale optische Zone 4' der Rückfläche jeweils einen sphärischen Krümmungsverlauf auf, der schematisch in Fig. 3b dargestellt ist. Bei diesem sphärischen Krümmungsverlauf ist der Krümmungsradius $r_{Sph}$ über den gesamten Durchmesser $d0_{Sph}$ der zentralen optischen Zone 4' konstant. Somit weisen die bekannten Kontaktlinsen eine Scheiteltiefe $s_{Sph}$ auf, die durch den Krümmungsradius $r_{Sph}$ und den Durchmesser $d0_{Sph}$ der zentralen optischen Zone 4' bestimmt ist. Für die Scheiteltiefe gilt in diesem Fall:

$$s_{Sph} = r_{Sph} - \sqrt{r_{Sph}^2 - (d0_{Sph}/2)^2} \, .$$

Nun wird der Krümmungsverlauf bei der dargestellten Ausführungsform der vorliegenden Erfindung anhand von den Fig. 3c und 3a beschrieben. In der dargestellten Ausführungsform weist die zentrale optische Zone 4 der Rückfläche einen elliptisch-oblongen Krümmungsverlauf auf. In Fig. 3a ist eine Ellipse dargestellt. Die Ellipse weist zwei Hauptachsen auf, eine prolonge Hauptachse 10 und eine oblonge Hauptachse 20. Die prolonge Hauptachse 10 erstreckt sich von dem Mittelpunkt M der Ellipse zu dem Hauptscheitel 11 und die oblonge Hauptachse 20 erstreckt sich von dem Mittelpunkt zu dem Nebenscheitel 21, wobei die oblonge Hauptachse 20 kürzer als die prolonge Hauptachse 10 ist. Die Krümmung der Ellipse in dem Nebenscheitel 21 entspricht der Krümmung eines Nebenscheitelkrümmungskreises NSK mit dem Radius $r_{NSK}$. Wie in Fig. 3a zu sehen ist, nimmt die Krümmung der Ellipsenoberfläche mit zunehmendem tangentialen Abstand von dem Nebenscheitel 21 zu und ist jeweils größer als die Krümmung des Nebenscheitelkrümmungskreises NSK. Dabei handelt es sich um einen elliptisch-oblongen Krümmungsverlauf.

In Fig. 3c ist schematisch die zentrale optische Zone 4 mit einem elliptisch-oblongen Krümmungsverlauf dargestellt. Die zentrale optische Zone 4 weist über ihren gesamten Durchmesser $d0_{Ellip}$ einen elliptisch-oblongen Krümmungsverlauf auf und hat in ihrem Nebenscheitel 21, der mit dem Zentrum Z der zentralen optischen Zone 4 zusammenfällt, einen Krümmungsradius $r_{Ellip\text{-}oblong}$, der dem Radius $r_{NSK}$ des Nebenscheitelkreises NSK entspricht. Die Scheiteltiefe $s_{Ellip}$ der zentralen optischen Zone 4 gemäß der Ausführungsform ist durch die Exzentrizität e der zugrundeliegenden Ellipse und den Durchmesser $d0_{Ellip}$ der zentralen optischen Zone 4 bestimmt. Die Exzentrizität der zugrundeliegenden Ellipse liegt dabei bevorzugt im Bereich zwischen -0,3 und -0,9. Für die Scheiteltiefe bei dem elliptisch-oblongen Verlauf gilt:

$$s_{Ellip} = a \cdot \left( 1 - \sqrt{1 - \left( d0_{Ellip} \Big/ 2b \right)^2} \right)$$

wobei $\quad q = \dfrac{-e^2}{1-e^2} \, ; \qquad a = \dfrac{r_{NSK}}{1-q} \qquad$ und

$$b = a \cdot \sqrt{1-q} \, .$$

Fig. 3d zeigt schematisch den sphärischen Krümmungsverlauf der zentralen optischen Zone 4' im Vergleich zu dem elliptisch-oblongen Krümmungsverlauf der zentralen optischen Zone gemäß der Ausführungsform. Dabei ist der Radius $r_{NSK}$ des Nebenscheitelkreises NSK der zentralen optischen Zone 4 gleich dem sphärischen Radius $r_{Sph}$ der zentralen optischen Zone 4' der bekannten Kontaktlinse gewählt ($r_{NSK} = r_{Sph}$) und die Durchmesser $d0_{Ellip}$ und $d0_{Sph}$ der zentralen optischen Zone 4 und der zentralen optischen Zone 4' sind identisch gewählt (d0$_{Sph}$ = d0$_{Ellip}$). In Fig. 3d ist zu sehen, dass die Scheiteltiefe s$_{Ellip}$ bei dem elliptisch-oblongen Krümmungsverlauf gegenüber der Scheiteltiefe s$_{Sph}$ bei dem sphärischen Krümmungsverlauf erhöht ist.

Die Darstellungen der Fig. 3b und 3c zeigen jeweils nur einen zweidimensionalen Schnitt durch die zentralen optischen Zonen 4' und 4, wohingegen die realen Kontaktlinsen selbstverständlich eine dreidimensional Erstreckung aufweisen. Gemäß der beschriebenen Ausführungsform weisen die zentralen optischen Zonen 4 und 4' bezüglich der zentralen optischen Achse X jeweils eine rotationssymmetrische Ausbildung auf. Je nach Anforderung kann die Rückfläche rotationssymmetrisch oder nicht-rotationssymmetrisch sein.

Die Vorderfläche 2 der Kontaktlinse 1 ist sphärisch oder asphärisch, rotationssymmetrisch, torisch oder aus einer Kombination der genannten Gestaltungen derart aufgebaut, dass sie zusammen mit der Rückfläche 3 die notwendige dioptrische Wirkung erzeugt, sodass sich bei dem Tragen für das Auge optimale Abbildungsverhältnisse ergeben.

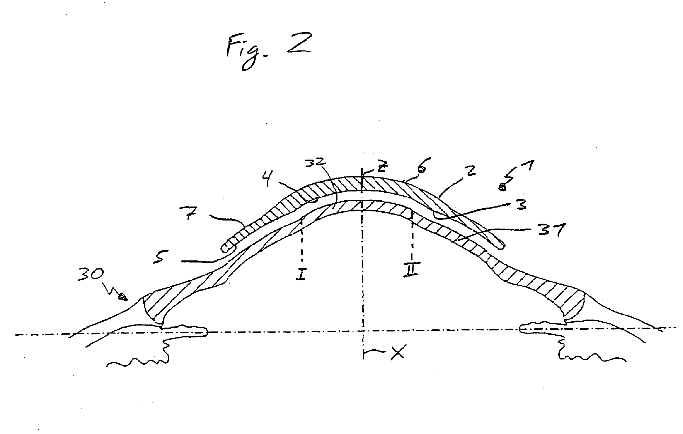

Im Folgenden wird anhand von Fig. 2 der Betrieb der beschriebenen Kontaktlinse in einem Ausführungsbeispiel erklärt.

Wie in Fig. 2 dargestellt ist, wird die Kontaktlinse 1 auf das Auge 30 des Kontaktlinsenträgers aufgesetzt. Die Hornhaut 31 weist in dem dargestellten Beispiel einen irregulär gekrümmten zentralen Abschnitt 32 zwischen den Linien I und II auf, der in Fig. 2 durch einen Keratokonus gegeben ist. Die zentrale optische Zone 4 überbrückt aufgrund ihres oblongen Krümmungsverlaufs den irregulär gekrümmten Abschnitt 32 und die Kontaktlinse 1 ruht mit dem peripheren Bereich 5 auf dem außerhalb des irregulär gekrümmten zentralen Abschnitt 32 befindlichen Bereich des Auges 30.

Aufgrund des oblongen Krümmungsverlaufs der zentralen optischen Zone 4 weist die Kontaktlinse 1 gegenüber einer Kontaktlinse, die für eine regulär gekrümmte Hornhaut gefertigt ist, keinen erhöhten Krümmungsradius im Bereich des Zentrums Z der zentralen optischen Zone 4 auf, sodass die Kontaktlinse in ihrer optischen Wirkung nicht stärker ausgelegt werden muss und die oben beschriebenen Nachteile der bekannten Kontaktlinsen für Keratokonus nicht auftreten. Dadurch, dass der irregulär gekrümmte Bereich 32 der Hornhaut 31 mit der Kontaktlinse 1 überbrückt werden kann, wird der mechanische Druck und damit die Belastung der veränderten Hornhaut vermindert und somit eine Verschlechterung des Zustands der Hornhaut vermieden.

Weitere Ausführungsformen und Abwandlungen

Obwohl in der obigen Ausführungsform ein elliptisch-oblonger Krümmungsverlauf der zentralen optischen Zone 4 beschrieben ist, kann die Kontaktlinse in dieser Zone auch einen anderen Krümmungsverlauf aufweisen. Dabei ist jedoch wichtig, dass kein sphärischer Krümmungsverlauf vorliegt, sondern ein asphärischoblonger Krümmungsverlauf. Das bedeutet, dass die zentrale optische Zone 4 in ihrem Zentrum Z die geringste Krümmung aufweist, also den größten Krümmungsradius, und die Krümmung mit zunehmendem Abstand von dem Zentrum Z zunimmt, also der Krümmungsradius kleiner wird, sodass verglichen mit einem sphärischen Krümmungsverlauf die Scheiteltiefe der zentralen optischen Zone 4 bei gleichem Durchmesser d0 und gleichem Krümmungsradius r$_z$ im Zentrum Z erhöht ist. Dabei weist die zentrale optische Zone 4 über ihre gesamte Fläche einen asphärischen Verlauf auf.

Bei diesem Krümmungsverlauf der zentralen optischen Zone 4 kann es sich z.B. wie oben beschrieben um einen elliptisch-oblongen Krümmungsradius oder um einen asphärischen Krümmungsverlauf höherer Ordnung handeln. Dabei sind die Rückfläche 3 und ihre zentrale optische Zone 4 in dem einfachsten Fall rotationssymmetrisch um die zentrale optische Achse X aufgebaut. Es kann jedoch ein anderer Krümmungsverlauf gewählt werden, der sich aus einer Kombination von rotationssymmetrischer, torischer, asymmetrischer, peripher exzentrischer und/oder quadrantendifferenter Gestaltung ergibt.

Der sich an die zentrale optische Zone 4 anschließende periphere Bereich 5 kann sphärisch mehrkurvig oder asphärisch gestaltet werden, sodass er der Kontur der Hornhaut optimal angepasst ist.

Die Gestaltung der zentralen optischen Zone mit einem hohen Maß an Symmetrie ist von Vorteil, falls dies bei der Geometrie des zu behandelnden Auges möglich ist, da dadurch ein optimales Abbildungsverhalten der Kontaktlinse erzielt werden kann.

**Patentansprüche**

1. Formstabile Kontaktlinse zur Korrektur bei irregulär gekrümmter Hornhaut mit einer Vorderfläche (2) und einer Rückfläche (3), die aus einer zentralen optischen Zone (4) und einer peripheren Zone (5) besteht, wobei
   die zentrale optische Zone (4) einen Durchmesser (d0) und ein Zentrum (Z) hat, durch das eine zentrale optische Achse (X) verläuft,
   **dadurch gekennzeichnet, dass** die Rückfläche der zentralen optischen Zone (4) um die zentrale optische Achse (X) derart asphärisch rotationssymmetrisch oder asphärisch nichtrotationssymmetrisch ausgebildet ist, dass bei gleichem Durchmesser (d0) und bei gleichem zentralen Krümmungsradius in dem Zentrum (Z) der zentralen optischen Zone (4) verglichen mit einer sphärischen optischen Zone eine erhöhte Scheiteltiefe der zentralen optischen Zone (4) gebildet ist.

2. Formstabile Kontaktlinse nach Anspruch 1, bei der die zentrale optische Zone (4) in ihrem Zentrum (Z)

die geringste Krümmung aufweist.

3. Formstabile Kontaktlinse nach Anspruch 1 oder 2, bei der die zentrale optische Zone (4) einen asphärisch-oblongen Krümmungsverlauf aufweist.

4. Formstabile Kontaktlinse nach einem der Ansprüche 1 bis 3, bei der der Krümmungsradius der zentralen optischen Zone (4) von dem Zentrum (Z) der zentralen optischen Zone zu deren Rand hin progressiv oder monoton abnimmt.

5. Formstabile Kontaktlinse nach einem der Ansprüche 1 bis 4, bei der die zentrale optische Zone (4) einen ellipsoiden Krümmungsverlauf aufweist.

6. Formstabile Kontaktlinse nach Anspruch 5, bei der eine Exzentrizität im Bereich zwischen -0.3 und -0.9 gegeben ist.

7. Formstabile Kontaktlinse nach einem der Ansprüche 1 bis 6, bei der die gesamte Rückfläche (3) rotationssymmetrisch, nichtrotationssymmetrisch, torisch, asymmetrisch, peripher exzentrisch, quadrantendifferent oder aus einer Kombination dieser Gestaltungen aufgebaut ist.

8. Formstabile Kontaktlinse nach einem der Ansprüche 1 bis 7, bei der die gesamte Rückfläche (3) rotationssymmetrisch aufgebaut ist.

9. Formstabile Kontaktlinse nach einem der Ansprüche 1 bis 8, bei der der periphere Bereich (5) sphärisch mehrkurvig oder asphärisch ausgebildet ist.

10. Formstabile Kontaktlinse nach einem der Ansprüche 1 bis 9, bei der der periphere Bereich (5) an die Kontur der zu versorgenden Hornhaut angepasst ist.

11. Formstabile Kontaktlinse nach einem der Ansprüche 1 bis 10, bei der die Vorderfläche (2) der Kontaktlinse entsprechend einer vorbestimmten dioptrischen Wirkung geformt ist.

12. Formstabile Kontaktlinse nach einem der Ansprüche 1 bis 11, bei der die Vorderfläche (2) sphärisch, asphärisch, rotationssymmetrisch, torisch oder aus einer Kombination dieser Gestaltungen aufgebaut ist.

**Claims**

1. Inherently stable contact lens for correcting an irregularly curved cornea, comprising a front surface (2) and a back surface (3) which consists of a central optical zone (4) and a peripheral zone (5), wherein the central optical zone (4) has a diameter (d0) and a center (Z), where a central optical axis (X) passes through,

**characterized in that**

the back surface of the central optical zone (4) is aspherically rotation-symmetrically or aspherically non-rotation-symmetrically formed around the central optical axis (X) such that an increased depth of an apex of the central optical zone (4) is formed in the center (Z) of the central optical zone (4) compared with a spherical optical zone with the same diameter (d0) and the same central radius of curvature.

2. Inherently stable contact lens according to claim 1, in which the central optical zone (4) has the smallest curvature in the center (Z) thereof.

3. Inherently stable contact lens according to claim 1 or 2, in which the central optical zone (4) comprises an aspherical-oblong course of curvature.

4. Inherently stable contact lens according to one of claims 1 to 3, in which the radius of curvature of the central optical zone (4) decreases from the center (Z) of the central optical zone to the boundary thereof in a progressive or monotone manner.

5. Inherently stable contact lens according to any of claims 1 to 4, in which the central optical zone (4) comprises an ellipsoid course of curvature.

6. Inherently stable contact lens according to claim 5, in which an eccentricity is provided in a range between -0,3 and -0,9.

7. Inherently stable contact lens according to any one of claims 1 to 6, in which the entire back surface (3) is formed rotation-symmetrically, non-rotation-symmetrically, torically, asymmetrically, peripheral eccentrically, different in the quadrants or of a combination of these configurations.

8. Inherently stable contact lens according to any one of claims 1 to 7, in which the entire back surface (3) is rotation-symmetrically formed.

9. Inherently stable contact lens according to any one of claims 1 to 8, in which the peripheral area (5) is spherically formed with multiple curves or aspherically formed.

10. Inherently stable contact lens according to any one of claims 1 to 9, in which the peripheral area (5) is adapted to the outline of the cornea to be cared.

11. Inherently stable contact lens according to any one of claims 1 to 10, in which the front surface (2) of the contact lens is formed in accordance to a predetermined dioptric effect.

**12.** Inherently stable contact lens according to any one of claims 1 to 11, in which the front surface (2) is formed spherically, aspherically, rotation-symmetrically, torically, or of a combination of these configurations.

## Revendications

**1.** Lentille de contact rigide pour corriger une cornée à courbure irrégulière, comprenant une face avant (2) et une face arrière (3), composée d'une zone optique centrale (4) et d'une zone périphérique (5),
dans laquelle la zone optique centrale (4) présente un diamètre (d0) et un centre (Z) passant par un axe optique central (X),
**caractérisée en ce que** la face arrière de la zone optique centrale (4) est réalisée autour de l'axe optique central (X) de manière asphérique avec symétrie de révolution ou de manière asphérique sans symétrie de révolution, de telle sorte que, pour un même diamètre (d0) et un même rayon de courbure central, en comparaison avec une zone optique sphérique, une profondeur de sommet accrue de la zone optique centrale (4) est formée au centre (Z) de la zone optique centrale (4).

**2.** Lentille de contact rigide selon la revendication 1, dans laquelle la zone optique centrale (4) présente le minimum de courbure en son centre (Z).

**3.** Lentille de contact rigide selon la revendication 1 ou 2, dans laquelle la zone optique centrale (4) présente un profil de courbure oblong asphérique.

**4.** Lentille de contact rigide selon l'une quelconque des revendications 1 à 3, dans laquelle le rayon de courbure de la zone optique centrale (4) diminue de manière progressive ou monotone du centre (Z) de la zone optique centrale jusqu'à son bord.

**5.** Lentille de contact rigide selon l'une quelconque des revendications 1 à 4, dans laquelle la zone optique centrale (4) présente un profil de courbure ellipsoïdal.

**6.** Lentille de contact rigide selon la revendication 5, dans laquelle il existe une excentricité dans la plage comprise entre -0,3 et -0,9.

**7.** Lentille de contact rigide selon l'une quelconque des revendications 1 à 6, dans laquelle la totalité de la face arrière (3) est constituée avec symétrie de révolution, sans symétrie de révolution, de manière torique, de manière asymétrique, de manière excentrique en périphérie, à quadrants différents ou à partir d'une combinaison de ces configurations.

**8.** Lentille de contact rigide selon l'une quelconque des revendications 1 à 7, dans laquelle la totalité de la face arrière (3) est constituée avec symétrie de révolution.

**9.** Lentille de contact rigide selon l'une quelconque des revendications 1 à 8, dans laquelle la zone périphérique (5) est réalisée de manière sphérique à courbes multiples ou de manière asphérique.

**10.** Lentille de contact rigide selon l'une quelconque des revendications 1 à 9, dans laquelle la zone périphérique (5) est adaptée au contour de la cornée à traiter.

**11.** Lentille de contact rigide selon l'une quelconque des revendications 1 à 10, dans laquelle la face avant (2) de la lentille de contact est formée selon un effet dioptrique prédéterminé.

**12.** Lentille de contact rigide selon l'une quelconque des revendications 1 à 11, dans laquelle la face avant (2) est constituée de manière sphérique, de manière asphérique, avec symétrie de révolution, de manière torique ou à partir d'une combinaison de ces configurations.

Fig. 7

Fig. 2

Fig. 3a)

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0235328 B1
- EP 0722573 B1
- WO 9507487 A

**In der Beschreibung aufgeführte Nicht-Patentliteratur**

- **ZADNIK, K. ; MUTTI, D.O.** *American Journal of Optometry and Physiological Optics,* September 1987, 698ff